# EUROPEAN PATENT APPLICATION

(11) **EP 0 713 855 A1**
(43) Date of publication of application: **29.05.1996**
(21) Application number: 95306814.5
(22) Date of filing: 27.09.1995
(51) Int. Cl.: C07C 59/125

(54) **Novel liquid fatty acid derivatives**

(30) Priority: 25.11.1994 JP 291016/94
(71) Applicant: NEW JAPAN CHEMICAL CO.,LTD., Kyoto-shi Kyoto-fu (JP)
(72) Inventor: Ueoka, Chiaki, Nara-shi, Nara-ken (JP); Kitagawa, Sachio, Yawata-shi, Kyoto-fu (JP)
(74) Representative: Cresswell, Thomas Anthony

(57) **Abstract**

A fatty acid derivative of formula (1) 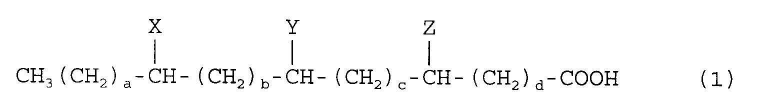
wherein each of X, Y and Z, which are the same or different, is a C₁-C₁₀ alkyloxy group or a C₃-C₁₀ cycloalkyloxy group, at least one of X, Y and Z being a C₁-C₁₀ alkyloxy group or a C₃-C₁₀ cycloalkyloxy group, each of a, b, c and d, which are the same or different, is an integer of 0 to 17 and the value of a + b + c + d is from 13 to 17; and salts thereof.

## Description

The present invention relates to novel liquid fatty acid derivatives. More specifically, the present invention relates to novel alkyloxy fatty acids, dialkyloxy fatty acids and trialkyloxy fatty acids.

Known liquid fatty acids include unsaturated fatty acids, branched-chain fatty acids, lower fatty acids and the like, and typical examples thereof are oleic acid, linoleic acid, linolenic acid and isostearic acid.

However, these liquid fatty acids can not always satisfy various commercial demands. It is thus commercially advantageous to broaden the selection range of liquid fatty acids for various new purposes.

For example, since conventional liquid fatty acids are not always satisfactory in oxidation stability and heat stability and emit an odor, there arises a problem that the selection range of these fatty acids is sometimes limited.

The present inventors conducted extensive research to overcome these problems and found that alkyloxy fatty acid derivatives having a specific structure are novel and useful compounds which are not disclosed in literature. The present invention has been accomplished based on this novel finding.

Thus, the present invention provides a liquid fatty acid derivative represented by the formula (1) 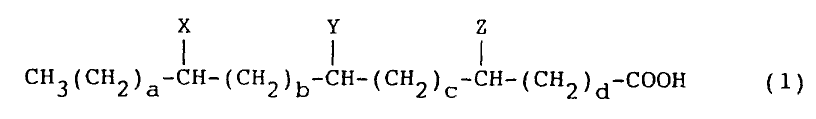 wherein X, Y and Z are the same or different and each represents a hydrogen atom, a C₁₋₁₀ alkyloxy group or a C₃₋₁₀ cycloalkyloxy group, at least one of X, Y and Z being a C₁₋₁₀ alkyloxy group or a C₃₋₁₀ cycloalkyloxy group; and a, b, c and d are the same or different and each represents an integer of 0 to 17, and the sum of a plus b plus c plus d is equal to 13 to 17.

More specific examples of the liquid fatty acid derivative represented by the formula (1) include monoalkyloxy fatty acids represented by the formula (2) 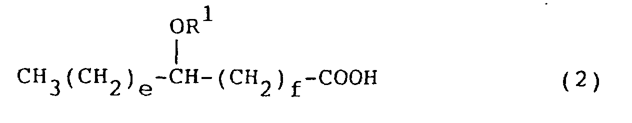 wherein R¹ represents a C₁₋₁₀ alkyl group or a C₃₋₁₀ cycloalkyl group, e and f are the same or different and e represents an integer of 0 to 19, f represents an integer of 1 to 19 and the sum of e plus f is equal to 15 to 19;
dialkyloxy fatty acids represented by the formula (3) 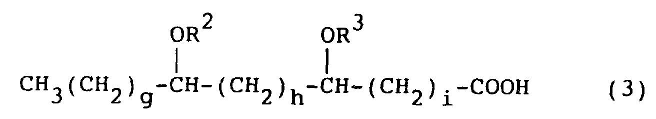 wherein R and R³ are the same or different and each represents a C₁₋₁₀ alkyl group or a C₃₋₁₀ cycloalkyl group, g, h and i are the same or different and each represents an integer of 0 to 18, and the sum of g plus h plus i is equal to 14 to 18; and
trialkyloxy fatty acids represented by the formula (4) 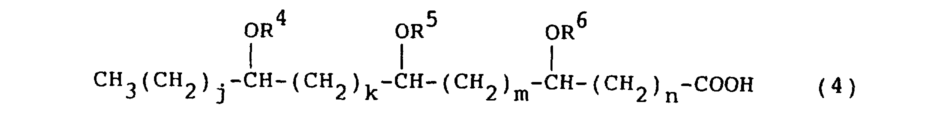 wherein R⁴, R⁵ and R⁶ are the same or different and each represents a C₁₋₁₀ alkyl group or a C₃₋₁₀ cycloalkyl group, j, k, m and n are the same or different and each represents an integer of 0 to 17, and the sum of j plus k plus m plus n is equal to 13 to 17.

Concerning the compounds of the formula (2), it is preferable that R¹ is a C₃₋₆ alkyl group or a C₅₋₇ cycloalkyl group, and e and f are the same or different and each represents an integer of 1 to 14.

As apparent from the above structural formulas, the liquid fatty acid derivatives of the present invention are alkyloxy-substituted fatty acids.

The alkyl or cycloalkyl groups which constitute the alkyloxy or cycloalkyloxy groups represented by X, Y or Z or which are represented by R¹ through R⁶ in the liquid fatty acid derivatives of the present invention include, for example, a straight- or branched-chain C₁₋₁₀ (preferably C₁₋₆) alkyl group such as methyl, ethyl, propyl, butyl and the like, and a C₃₋₁₀ (preferably C₅₋₇) cycloalkyl group such as cyclopentyl, cyclohexyl and the like.

Among the foregoing liquid fatty acids of the present invention, preferable compounds include, for example, those represented by the formula (2-p) 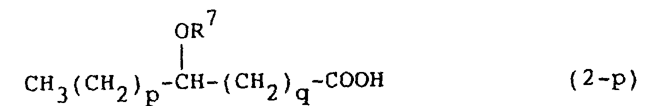 wherein R⁷ is a C₃₋₆ alkyl group or a C₅₋₇ cycloalkyl group, and p and q are the same or different and each represents an integer of 1 to 14, and the sum of p plus q is equal to 15 to 19.

Typical examples of the monoalkyloxy fatty acids represented by the formula (2) or (2-p) include 9-methoxystearic acid, 9-ethoxystearic acid, 9-n-propyloxystearic acid, 9-iso-propyloxystearic acid, 9-n-butoxystearic acid, 9-tert-butoxystearic acid, 9-cyclohexyloxystearic acid, 10-methoxystearic acid, 10-ethoxystearic acid, 10-n-propyloxystearic acid, 10-isopropyloxystearic acid, 10-n-butoxystearic acid, 10-tert-butoxystearic acid, 10-cyclohexyloxystearic acid, 6-methoxystearic acid, 7-methoxystearic acid, 11-methoxystearic acid, 12-methoxystearic acid, 9-methoxyeicosanoic acid, 10-methoxyeicosanoic acid, 11-methoxyeicosanoic acid, 12-methoxyeicosanoic acid, 11-methoxybehenic acid, 12-methoxybehenic acid, 13-methoxybehenic acid, 14-methoxybehenic acid, and the like.

Typical examples of the dialkyloxy fatty acids represented by the formula (3) include 9,12-dimethoxystearic acid, 10,13-dimethoxystearic acid, 9,10-dimethoxystearic acid, 9,13-dimethoxystearic acid, 10,12-dimethoxystearic acid, 10,13-dimethoxystearic acid, 11,12-dimethoxystearic acid, 11,13-dimethoxystearic acid and the like.

Typical examples of the trialkyloxy fatty acids represented by the formula (4) include 6,9,12-trimethoxystearic acid, 6,9,13-trimethoxystearic acid, 6,10,12-trimethoxystearic acid, 6,10,13-trimethoxystearic acid, 7,9,12-trimethoxystearic acid, 7,9,13-trimethoxystearic acid, 7,10,12-trimethoxystearic acid, 7,10,13-trimethoxystearic acid, 9,12,15-trimethoxystearic acid, 9,12,16-trimethoxystearic acid, 9,13,15-trimethoxystearic acid, 9,13,16-trimethoxystearic acid, 10,12,15-trimethoxystearic acid, 10,12,16-trimethoxystearic acid, 10,13,15-trimethoxystearic acid, 10,13,16-trimethoxystearic acid, 8,11,14-trimethoxyeicosanoic acid, 8,11,15-trimethoxyeicosanoic acid, 8,12,14-trimethoxyeicosanoic acid, 8,12,15-trimethoxyeicosanoic acid, 9,11,14-trimethoxyeicosanoic acid, 9,11,15-trimethoxyeicosanoic acid, 9,12,14-trimethoxyeicosanoic acid, 9,12,15-trimethoxyeicosanoic acid, 7,10,13-trimethoxybehenic acid, 7,10,14-trimethoxybehenic acid, 7,11,13-trimethoxybehenic acid, 7,11,14-trimethoxybehenic acid, 8,10,13-trimethoxybehenic acid, 8,10,14-trimethoxybehenic acid, 8,11,13-trimethoxybehenic acid, 8,11,14-trimethoxybehenic acid, and the like.

The fatty acid derivatives of the present invention can be produced by various methods, among which the following two methods are especially advantageous.

### (A) Oxymercuration-demercuration reaction

The fatty acid derivatives of the present invention can be produced, for example, by oxymercuration-demercuration reaction. That is to say, the desired alkyloxy fatty acid can be produced by reacting a prescribed unsaturated fatty acid alkyl or cycloalkyl ester with an alcohol in the presence of a Hg (II) compound at 0 to 100°C, preferably 20 to 50°C, for 0.5 to 8 hours, preferably 1 to 3 hours, followed by reduction with a reducing agent such as sodium borohydride at 0 to 100°C, preferably 20 to 50°C, for 0.5 to 8 hours, preferably 1 to 3 hours.

Specifically, the liquid fatty acid derivative of the present invention represented by the formula (1) is produced by subjecting (i) an alkyl or cycloalkyl ester of an unsaturated fatty acid represented by the formula (1a)

CH₃(CH₂)ᵣ-Eₓ-(CH₂)ₛ-E_{y}-(CH₂)ₜ-E_{z}-(CH₂)ᵤ-COOH (1a)

wherein E is a group -CH=CH-, x, y and z are the same or different and each represents 0 or 1, at least one of x, y and z being 1, and r, s, t and u are the same or different and each represents an integer of 0 to 18, and the sum of r plus s plus t plus u is equal to 10 to 18 and (ii) an alcohol represented by the formula (5)

ROH (5)

wherein R is a C₁₋₁₀ alkyl group or a C₃₋₁₀ cycloalkyl group to an oxymercuration-demercuration reaction under the foregoing conditions.

Usable as the alkyl or cycloalkyl ester of the unsaturated fatty acid of the formula (1a) are esters of the unsaturated fatty acid of the formula (1a) with an aliphatic alcohol having about 1 to 10 carbon atoms, preferably 1 to 6 carbon atoms or with an alicyclic alcohol having 5 to 7 carbon atoms. Such alkyl or cycloalkyl esters are known and readily available or easily produced by a known method. Specific examples thereof include various compounds, among which the following compounds are preferably used.
(i) Compounds having one C-C double bond
   methyl oleate, ethyl oleate, propyl oleate, butyl oleate, cyclohexyl oleate, methyl elaidate, ethyl elaidate, propyl elaidate, butyl elaidate, cyclohexyl elaidate, methyl 6-octadecenoate, ethyl 6-octadecenoate, propyl 6-octadecenoate, butyl 6-octadecenoate, cyclohexyl 6-octadecenoate, methyl 11-octadecenoate, ethyl 11-octadecenoate, propyl 11-octadecenoate, butyl 11-octadecenoate, cyclohexyl 11-octadecenoate, methyl 9-eicosenoate, ethyl 9-eicosenoate, propyl 9-eicosenoate, butyl 9-eicosenoate, cyclohexyl 9-eicosenoate, methyl 11-eicosenoate, ethyl 11-eicosenoate, propyl 11-eicosenoate, butyl ll-eicosenoate, cyclohexyl 11-eicosenoate, methyl 11-docosenoate, ethyl 11-docosenoate, propyl 11-docosenoate, butyl 11-docosenoate, cyclohexyl 11-docosenoate, methyl 13-docosenoate, ethyl 13-docosenoate, propyl 13-docosenoate, butyl 13-docosenoate, cyclohexyl 13-docosenoate, methyl 2-octadecenoate, ethyl 2-octadecenoate, propyl 2-octadecenoate, butyl 2-octadecenoate, cyclohexyl 2-octadecenoate and the like.
(ii) Compounds having two C-C double bonds
   methyl linoleate, ethyl linoleate, propyl linoleate, butyl linoleate, cyclohexyl linoleate, methyl 10,12-octadecadienoate, ethyl 10,12-octadecadienoate, propyl 10,12-octadecadienoate, butyl 10,12-octadecadienoate, cyclohexyl 10,12-octadecadienoate, and the like.
(iii) Compounds having three C-C double bonds
   methyl linolenate, ethyl linolenate, propyl linolenate, butyl linolenate, cyclohexyl linolenate, methyl γ-linolenate, ethyl γ-linolenate, propyl γ-linolenate, butyl γ-linolenate, cyclohexyl γ-linolenate, methyl eleostearate, ethyl eleostearate, propyl eleostearate, butyl eleostearate, cyclohexyl eleostearate, methyl 8,11,14-eicosatrienoate, ethyl 8,11,14-eicosatrienoate, propyl 8,11,14-eicosatrienoate, butyl 8,11,14-eicosatrienoate, cyclohexyl 8,11,14-eicosatrienoate, and the like.

Generally, an alkyl or cycloalkyl ester of the acid of the formula (1a) having one C-C double bond is used for preparing monoalkyloxy fatty acids of the formula (2), and an alkyl or cycloalkyl ester of the acid of the formula (1a) having two C-C double bonds is used for preparing dialkyloxy fatty acids of the formula (3), and an alkyl or cycloalkyl ester of the acid of the formula (1a) having three C-C double bonds is used for preparing trialkyloxy fatty acids of the formula (4).

The alcohol of the formula (5) includes a C₁₋₁₀ (preferably C₁₋₆) saturated aliphatic alcohol such as methanol, ethanol, propanol, butanol, pentanol, hexanol, heptanol, octanol, nonanol, decanol and the like, and a C₃₋₁₀ (preferably C₅₋₇) saturated alicyclic alcohol such as cyclopropanol, cyclobutanol, cyclopentanol, cyclohexanol, cycloheptanol, cyclooctanol, cyclononanol, cyclodecanol and the like.

A mixture of these alcohols is used for preparing di- or trialkyloxy fatty acid of the formula (3) or (4) wherein one or two alkoxy groups is/are different from the other alkoxy group(s).

The amount of the alcohol of the formula (5) to be used in the reaction is 1 to 1000 moles, preferably 10 to 100 moles, per mole of the alkyl or cycloalkyl ester of the unsaturated fatty acid of the formula (1a).

Preferable examples of the Hg (II) compound are mercury acetate and mercury trifluoroacetate. The amount of the Hg (II) compound is 1 to 20 equivalents, preferably 1 to 5 equivalents, per mole of the alkyl or cycloalkyl ester of the unsaturated aliphatic fatty acid of the formula (1a).

The reaction of the alkyl or cycloalkyl ester of the unsaturated fatty acid of the formula (1a) with the alcohol of the formula (5) in the presence of the Hg (II) compound is carried out using an excess amount of the alcohol of the formula (5). Therefore, the alcohol generally serves also as an solvent. The reaction is preferably carried out at atmospheric pressure in an inert gas (e.g. nitrogen) atmosphere.

On completion of the reaction of the alkyl or cycloalkyl ester of the unsaturated fatty acid of the formula (1a) with the alcohol of the formula (5), the obtained reaction mixture is subjected to reduction by addition of a reducing agent such as sodium borohydride.

The reduction is carried out under alkaline conditions by adding to the reaction mixture an aqueous solution of an alkali metal hydroxide such as sodium hydroxide, potassium hydroxide and the like. The alkali metal hydroxide is used in an amount of about 1 to 50 moles, preferably 2 to 10 moles, per mole of the Hg(II) compound.

The amount of the reducing agent such as sodium borohydride can be suitably selected from a wide range, but generally range from about 0.01 to 50 moles, preferably from about 0.1 to 2 moles, per mole of the Hg(II) compound.

This reduction reaction is preferably carried out at atmospheric pressure.

### (B) A method using a hydroxy-containing saturated fatty acid

The fatty acid derivatives of the formula (1) of the present invention can also be produced by the following method instead of the above-mentioned method (A). Thus, an alkyl or cycloalkyl ester of a hydroxy-containing saturated fatty acid represented by the formula (1b) 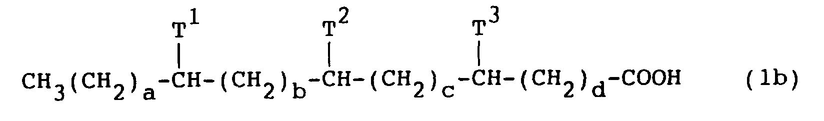 wherein T¹, T and T³ are the same or different and each represents a hydrogen atom or a hydroxyl group, and at least one of T¹, T and T³ must be a hydroxyl group, and a, b, c and d are as defined in the formula (1) is treated with an alkali metal hydride or an alkaline earth metal hydride such as sodium hydride, calcium hydride and the like in an inert polar organic solvent such as dimethylformamide, dimethylacetamide, N-methylpyrrolidone, dimethylsulfoxide, acetonitrile, dioxane, tetrahydrofuran, tetrahydropyran, diglyme and the like at about 0 to 150 °C for about 0.1 to 30 hours at atmospheric pressure, and then an alkyl iodide or cycloalkyl iodide represented by the formula (6)

R-I (6)

wherein R is a C₁₋₁₀ alkyl group or a C₃₋₁₀ cycloalkyl group is added to the resulting reaction mixture, after which a reaction is carried out at about 0 to 150 °C for about 0.1 to 30 hours at atmospheric pressure.

The hydroxy-containing saturated fatty acids of the formula (1b) are known and readily available or easily produced by a known method. Examples of such hydroxy-containing saturated fatty acids include 12-hydroxystearic acid, 9-hydroxyoctadecanoic acid, 10-hydroxyoctadecanoic acid, 9,10-dihydroxyoctadecanoic acid and the like. The fatty acid of the formula (1b) is used in the form of an alkyl or cycloalkyl ester, particularly C₁₋₁₀ (preferably C₁₋₆) alkyl ester. Such alkyl or cycloalkyl esters are known or readily prepared by conventional methods.

The compound of the formula (6) includes, for example, a straight- or branched-chain C₁₋₁₀ (preferably C₁₋₆) alkyl iodide such as methyl iodide, ethyl iodide, propyl iodide, butyl iodide and the like, and a C₃₋₁₀ (preferably C₅₋₇) cycloalkyl iodide such as cyclopentyl iodide, cyclohexyl iodide and the like.

A mixture of these compounds of the formula (6) is used for preparing di- or trialkyloxy fatty acid of the formula (3) or (4) wherein one or two alkyloxy groups is/are different from the other alkyloxy group(s).

The alkali metal hydride or alkaline earth metal hydride is usually used in an amount of 1 to 5 moles per mole of the alkyl or cycloalkyl ester of the hydroxy-containing saturated fatty acid of the formula (1b).

The compound of the formula (6) is usually used in an amount of 1 to 100 moles, preferably 1 to 20 moles, per mole of the alkyl or cycloalkyl ester of the hydroxy-containing saturated fatty acid of the formula (1b).

An alkyloxylated saturated fatty acid ester obtained by the above method (A) or (B) is separated and purified by a known separation and purification method such as chromatography and the like. The purified product is then converted to the desired fatty acid derivative of the invention by a conventional hydrolysis method using an alkali metal hydroxide or the like such as sodium hydroxide, potassium hydroxide, etc. The hydrolysis can be carried out under conditions conventionally employed, typically and preferably in a mixture containing water, an ether such as tetrahydrofuran and/or a lower alcohol such as methanol under reflux for about 0.1 to 10 hours.

In isolating the alkyloxy fatty acid of the present invention, the reaction mixture obtained by said method (A) may be subjected to the isolation and purification step as mentioned above, but it is generally preferable that the desired alkyloxy fatty acid of the present invention is isolated by first neutralizing the reaction mixture obtained by said method (A) by addition of an acid such as hydrochloric acid to obtain an alkyloxy fatty acid, esterifying the alkyloxy fatty acid with a lower alcohol in a conventional manner, isolating the resulting ester by means of high performance liquid chromatography or some other means and finally hydrolyzing the isolated ester to convert it to the desired alkyloxy fatty acid.

The various alkyloxy fatty acids of the formula (1) according to the present invention may be converted into salts. Conventional salification techniques may be used. Suitable salts include ammonium salts and salts with alkali metals and alkaline earth metals, for instance sodium, potassium, caesium, magnesium and calcium, preferably sodium or potassium.

The fatty acids of formula (I) are novel liquid fatty acid derivatives useful as cosmetic materials, materials for lubricants and materials for various esters.

For example, alkali metal salts of the alkoxy fatty acid of the invention obtained by neutralizing the alkyloxyfatty acid with an alkali metal hydroxide (e.g. NaOH, KOH) or with an alkali metal carbonate (e.g. Na₃CO₃, K₂CO₃) in a conventional manner, monoethanolamide of the alkyloxy fatty acid of the invention obtained by the method described in J. Am. Oil Chem. Soc., 47, 91 (1970) or diethanolamide of the alkyloxy fatty acid of the invention obtained by the method described in J. Am. Oil Chem. Soc., 34, 323 (1957), and sugar esters (e.g. sucrose esters, glucose esters) of the alkyloxy fatty acid of the invention obtained by the method described in J. Am. Oil Chem. Soc., 49, 524 (1972) are useful as an additive to be added to soaps, shampoos, rinses, facial cleaners, cosmetic creams, lotions or the like for improving stability at low temperature, conditioning actions or moisture retention or for inhibiting skin irritation. When the above alkali metal salts, mono- or diethanolamide or sugar ester are used for preparing, or added to, soaps, shampoos, cleansing preparations or like cleansing compositions, the resulting products are excellent in foaming properties, foam stability or a rinsability.

Further, alkyl esters (particularly C₆₋₂₀ alkyl ester) of the alkyloxy fatty acid of the invention obtained by the conventional esterification method in an excess amount of an alcohol in the presence of an acid catalyst (e.g., hydrochloric acid, sulfuric acid, p-toluenesulfonic acid) or mono-, di- or triglycerides of the alkyloxy fatty acid of the invention obtained by the method described in Chem. Rev. 58, 845 (1958) are useful as an additive to be added to lubricants (e.g., refrigerating machine oils, turbine oils, engine oils, gear oils, machine oils, bearing oils, heat treating oils, cutting oils, rust preventive oils, insulating oils, greases, liquid paraffins, etc.) for improving flow property at low temperature, oxidation stability, heat stability or lubricating properties.

The alkyloxy fatty acid derivatives of the present invention are novel and useful liquid fatty acids whose important characteristics are excellence in oxidation stability and heat stability and freedom from an odor. For example, of the conventional liquid fatty acids, unsaturated acids such as oleic acid are easily deteriorated by oxidation or by heat and also emit an odor. On the other hand, the alkyloxy fatty acids of the present invention are resistant to oxidation or heat and do not emit an odor. Further, isostearic acid which is also a conventional liquid fatty acid is only obtained as a by-product of dimer acid and is therefore unsatisfactory from the standpoint of stable supply. On the other hand, the alkyloxy fatty acids of the present invention are prepared by chemical synthesis and are therefore satisfactory from the standpoint of stable supply.

The fatty acid derivatives of the present invention can be satisfactorily used for the applications wherein the conventional liquid fatty acids are scarcely usable due to their insufficiency of oxidation stability and heat stability and emission of an odor.

### Examples

The following examples illustrate the present invention in further detail.

### Example 1

A 200 ml, four-necked flask equipped with a thermometer, a gas inlet and a stirrer was charged with 5.0 g (17 mmoles) of methyl oleate, 5.4 g (17 mmoles) of mercury (II) acetate and 40 ml of methanol, and the mixture was stirred under a nitrogen atmosphere at room temperature for 1.5 hours.

A 20 ml quantity of a 3N aqueous solution of sodium hydroxide and then 20 ml of an aqueous solution of 0.5M sodium borohydride in a 3N aqueous solution of sodium hydroxide were successively added, followed by stirring at room temperature for 1.5 hours.

After completion of the reaction, the reaction mixture was filtered, and the filtrate was neutralized with a dilute aqueous solution of hydrochloric acid and subjected to extraction with ether. The extract was then washed twice with a saturated aqueous solution of sodium chloride. After evaporating the solvent, 80 ml of methanol and 0.01 g of p-toluenesulfonic acid were added and the mixture was refluxed for 2 hours in order to convert the alkyloxy fatty acid to the methyl ester.

Methyl 9-methoxystearate was isolated using a high pressure liquid chromatography for separation, and 1.5 g of sodium hydroxide, 15 ml of tetrahydrofuran, 30 ml of methanol and 1 ml of water were added thereto. The mixture was refluxed for 1.5 hours for hydrolysis, giving 9-methoxystearic acid. The yield was 42.0%.

The characteristic values of the obtained product were as follows, whereby the structure of the formula (2) wherein R¹=CH₃ is supported.
IR (NaCl, cm⁻¹):
   3300 - 2500 (OH), 2928 (CH), 2856 (CH), 1711 (C=O), 1465, 1377, 1197, 1098 (C-O-C), 938
¹NMR (CDCl₃, δ ppm)
   11.1 (1H, brs, OH), 3.22 (3H, s, OCH₃), 3.13 (1H, m, OCH), 2.35 (2H, t, CH₂CO₂H), 1.64 (2H, m, CH₂), 1.38-1.51 (4H, brm, CH₂ x 2), 1.22 - 1.38 (22H, brm, CH₂ x 11), 0.89 (3H, t, CH₃)
melting point: 1.6°C, cloud point: -43°C

### Example 2

The procedure of Example 1 was repeated with the exception that ethanol was used in lieu of methanol and that the reaction of methyl oleate with the alcohol was carried out at 30°C. The yield of 9-ethoxystearic acid was 39.2%.

The characteristic values of the obtained product were as follows, whereby the structure of the formula (2) wherein R¹=CH₃CH₂ is supported.
IR(NaCl, cm⁻¹):
   3300 - 2500 (OH), 2927 (CH), 2856 (CH), 1713 (C=O), 1466, 1413, 1373, 1343, 1285, 1111 (C-O-C), 939
¹NMR (CDCl₃, δ ppm)
   11.1 (1H, brs, OH), 3.48 (2H, q, OCH₂), 3.20 (1H, m, OCH), 2.35 (2H, t, CH₂CO₂H), 1.64 (2H, m, CH₂), 1.38-1.51 (4H, brm, CH₂ x 2), 1.22 - 1.38 (22H, brm, CH₂ x 11), 1.19 (3H, t, OCH₂CH₃), 0.89 (3H, t, CH₃)
melting point: -0.7°C, cloud point: -50°C

### Example 3

The procedure of Example 2 was repeated with the exception that n-propanol was used in lieu of ethanol and that the reaction of methyl oleate with the alcohol was carried out for 4 hours. The yield of 10-n-propyloxystearic acid was 37.6%.

The characteristic values of the obtained product were as follows, whereby the structure of the formula (2) wherein R¹-CH₃(CH₂)₂ is supported.
IR (NaCl, cm⁻¹):
   3300 - 2500 (OH), 2927 (CH), 2856 (CH), 1713 (C=O), 1465, 1377, 1284, 1090 (C-O-C), 938
¹NMR (CDCl₃, δ ppm)
   10.6 (1H, brs, OH), 3.37 (2H, t, OCH₂), 3.19 (1H, m, OCH), 2.35 (2H, t, CH₂CO₂H), 1.63 (2H, m, CH₂), 1.57 (2H, m, OCH₂CH₂), 1.38 - 1.51 (4H, brm, CH₂ x 2), 1.22 - 1.38 (22H, brm, CH₂ x 11), 0.93 (3H, t, CH₃), 0.89 (3H, t, CH₃)
melting point: -49.0°C, cloud point: -53°C

### Example 4

The procedure of Example 3 was repeated with the exception that n-butanol was used in lieu of n-propanol. The yield of 10-n-butoxystearic acid was 35.6%.

The characteristic values of the obtained product were as follows, whereby the structure of the formula (2) wherein R¹=CH₃(CH₂)₃ is supported.
IR(NaCl, cm⁻¹):
   3300 - 2500 (OH), 2928 (CH), 2856 (CH), 1712 (C=O), 1466, 1378, 1285, 1096 (C-O-C), 937
¹NMR (CDCl₃, δ ppm)
   10.8 (1H, brs, OH), 3.41 (2H, t, OCH₂), 3.18 (1H, m, OCH), 2.35 (2H, m, CH₂CO₂H), 1.64 (2H, m, CH₂), 1.54 (2H, m, OCH₂CH₂), 1.22 - 1.51 (28H, brm, CH₂ x 14), 0.92 (3H, t, CH₃), 0.89 (3H, t, CH₃)
melting point: -23.1°C, cloud point: -60°C

### Example 5

The procedure of Example 4 was repeated with the exception that tert-butanol was used in lieu of n-butanol and that 6.3 g (17 mmoles) of mercury (II) trifluoroacetate was used in lieu of mercury (II) acetate. The yield of 10-tert-butoxystearic acid was 35.4%.

The characteristic values of the obtained product were as follows, whereby the structure of the formula (2) wherein R¹=C(CH₃)₃ is supported.
IR(NaCl, cm⁻¹):
   3300 - 2500 (OH), 2928 (CH), 2856 (CH), 1712 (C=O), 1464, 1392, 1367, 1285, 1096 (C-O-C), 937
¹NMR (CDCl₃, δ ppm)
   10.8 (1H, brs, OH), 3.15 (1H, m, OCH), 2.35 (2H, m, CH₂CO₂H), 1.64 (2H, m, CH₂), 1.38 - 1.51 (4H, brm, CH₂ x 2), 1.22 - 1.38 (22H, brm, CH₂ x 11), 1.20 (9H, s, CH₃ x 3), 0.89 (3H, t, CH₃)
melting point: -25.3°C, cloud point: -58°C

### Example 6

The procedure of Example 1 was repeated with the exception that 6.0 g (17 mmoles) of methyl erucate was used in lieu of methyl oleate. The yield of 13-methoxybehenic acid was 40.1%.

The characteristic values of the obtained product were as follows, whereby the structure of the formula (2) wherein R¹=CH₃ is supported.
IR (NaCl, cm⁻¹):
   3300 - 2500 (OH), 2928 (CH), 2856 (CH), 1712 (C=O), 1464, 1375, 1197, 1099 (C-O-C), 938
¹NMR (CDCl₃, δ ppm):
   11.1 (1H, brs, OH), 3.22 (3H, s, OCH₃), 3.15 (1H, m, OCH), 2.35 (2H, t, CH₂CO₂H), 1.64 (2H, m, CH₂), 1.38-1.51 (4H, brm, CH₂ x 2), 1.22 - 1.38 (30H, brm, CH₂ x 15), 0.89 (3H, t, CH₃)
melting point: 12.3°C

### Example 7

The procedure of Example 1 was repeated with the exception that methyl linoleate was used in lieu of methyl oleate and the amounts of mercury acetate (II) and methanol were increased to 10.8 g (34 mmoles) and 70 ml, respectively. The yield of 9,12-dimethoxystearic acid was 39.0 %.

The characteristic values of the obtained product were as follows, whereby the structure of the formula (3) wherein R=R³=CH₃ is supported.
IR (NaCl, cm-1):
   3300 - 2500 (OH), 2929 (CH), 2856 (CH), 1711 (C=O), 1466, 1376, 1199, 1098 (C-O-C), 939
¹NMR (CDCl₃, δ ppm):
   11.1 (1H, brs, OH), 3.22 (6H, s, OCH₃ x 2), 3.13 (2H, m, OCH x 2), 2.35 (2H, t, CH₂CO₂H), 1.64 (2H, m, CH₂), 1.38 - 1.52 (8H, brm, CH₂ x 4), 1.22 - 1.38 (16H, brm, CH₂ x 8), 0.89 (3H, t, CH₃)
melting point: -32.8°C

### Example 8

The procedure of Example 1 was repeated with the exception that methyl linolenate was used in lieu of methyl oleate and the amounts of mercury acetate (II) and methanol were increased to 16.2 g (51 mmoles) and 100 ml, respectively. The yield of 9,12,15-trimethoxystearic acid was 36.1%.

The characteristic values of the obtained product were as follows, whereby the structure of the formula (4) wherein R⁴=R⁵=R⁶=CH₃ is supported.
IR (NaCl, cm⁻¹):
   3300 - 2500 (OH), 2927 (CH), 2856 (CH), 1710 (C=O), 1465, 1378, 1200, 1099 (C-O-C), 938
¹NMR (CDCl₃, δ ppm):
   11.1 (1H, brs, OH), 3.22 (9H, s, OCH₃ x 3), 3.13 (3H, m, OCH x 3), 2.35 (2H, t, CH₂CO₂H), 1.64 (2H, m, CH₂), 1.36 - 1.52 (12H, brm, CH₂ x 6), 1.22 - 1.36 (10 H, brm, CH₂ x 5), 0.89 (3H, t, CH₃)
melting point: -41.5°C

### Example 9

The procedure of Example 5 was repeated with the exception that cyclohexanol was used in lieu of tert-butanol. The yield of 9-cyclohexyloxystearic acid was 37.1%.

The characteristic values of the obtained product were as follows, whereby the structure of the formula (2) wherein R¹=cyclohexyl is supported.
formula (2) wherein R¹=cyclohexyl is supported. IR(NaCl, cm⁻¹):
   3300 - 2500 (OH), 2927 (CH), 2855 (CH), 1714 (C=O), 1465, 1374, 1285, 1095 (C-O-C), 939
¹NMR(CDCl₃, δ ppm)
   11.1 (1H, brs, OH), 3.25 (1H, m, OCH), 3.19 (1H, m, OCH), 2.35 (2H, t, CH₂CO₂H), 1.63 (2H, m, CH₂), 1.38-1.51 (4H, brm, CH₂ x 2), 1.22 - 1.38 (32H, brm, CH₂ x 16), 0.89 (3H, t, CH₃)
melting point: -24.7°C, cloud point: -62°C

### Example 10

A 100 ml, four-necked flask equipped with a thermometer, a gas inlet and a stirrer was charged with 5 g (16 mmoles) of methyl 12-hydroxystearate, 0.6 g (16 mmoles) of 60% sodium hydride which had been washed with n-hexane, 20 ml of dimethylformamide which had been dried over calcium hydride and distilled. The mixture was stirred under a nitrogen atmosphere at room temperature for 3 hours.

Then, 5 ml of ethyl iodide was added, followed by stirring for further 6 hours.

After completion of the reaction, the reaction mixture was neutralized with a dilute aqueous solution of hydrochloric acid and subjected to extraction with ether. The extract was then washed twice with a saturated aqueous solution of sodium chloride. After evaporating the solvent, methyl 12-ethoxystearate was isolated by silica gel column chromatography (2.7 cm in diameter x 43 cm in length, eluent (volume ratio): n-hexane/ethyl acetate = 20/1). Then, 1.5 g of sodium hydroxide, 15 ml of tetrahydrofuran, 30 ml of methanol and 1 ml of water were added thereto. The mixture was refluxed for 1.5 hours for hydrolysis, giving 12-ethoxystearic acid. The yield was 76.2%.

The characteristic values of the obtained product were as follows, whereby the structure of the formula (2) wherein R¹=CH₃CH₂ is supported.
IR (NaCl, cm⁻¹):
   3300 - 2500 (OH), 2927 (CH), 1713 (C=O), 1466, 1373, 1284, 1111 (C-O-C), 939
¹NMR (CDCl₃, δ ppm):
   11.0 (1H, brs, OH), 3.48 (2H, q, OCH₂), 3.21 (1H, quint, CH), 2.35 (2H, t, CH₂CO₂H), 1.64 (2H, quint, CH₂CH₂CO₂H), 1.5 - 1.2 (26H, brm, CH₂ x 13), 1.20 (3H, t, OCH₂CH₃), 0.90 (3H, t, CH₃)
melting point: 13.5°C

### Example 11

The procedure of Example 10 was repeated with the exception that n-propyl iodide was used in lieu of ethyl iodide. The yield of 12-n-propoxystearic acid was 71.8%.

The characteristic values of the obtained product were as follows, whereby the structure of the formula (2) wherein R¹=CH₃(CH₂)₂ is supported.
IR (NaCl, cm⁻¹):
   3300 - 2500 (OH), 2928 (CH), 2856 (CH), 1713 (C=O), 1465, 1377, 1283, 1091 (C-O-C), 939
¹NMR (CDCl₃, δ ppm):
   10.9 (1H, brs, OH), 3.37 (2H, t, OCH₂), 3.20 (1H, quint, CH), 2.35 (2H, t, CH₂CO₂H), 1.64 (2H, quint, CH₂CH₂CO₂H), 1.57 (2H, m, OCH₂CH₂), 1.5 - 1.2 (26H, brm, CH₂ x 13), 0.93 (3H, t, O(CH₂)₂CH₃), 0.89 (3H, t, CH₃)
melting point: -11.9°C

### Example 12

The procedure of Example 10 was repeated with the exception that n-butyl iodide was used in lieu of ethyl iodide. The yield of the obtained 12-n-butoxystearic acid was 70.1%.

The characteristic values of the obtained product were as follows, whereby the structure of the formula (2) wherein R¹=CH₃(CH₂)₃ is supported.
IR (NaCl, cm⁻¹):
   3300 - 2500 (OH), 2927 (CH), 2856 (CH), 1713 (C=O), 1466, 1378, 1284, 1097 (C-O-C), 940
¹NMR (CDCl₃, δ ppm):
   11.1 (1H, brs, OH), 3.41 (2H, t, OCH₂), 3.19 (1H, quint, CH), 2.35 (2H, t, CH₂CO₂H), 1.64 (2H, quint, CH₂CH₂CO₂H), 1.54 (2H, quint, OCH₂CH₂), 1.5 - 1.2 (28H, brm, CH₂ x 14), 0.92 (3H, t, O(CH₂)₃CH₃), 0.89 (3H, t, CH₃)
melting point: -6.9°C

### Example 13

The procedure of Example 3 was repeated with the exception that methyl 2-octadecenoate was used in lieu of methyl oleate.

After completion of the reaction, the reaction mixture was filtered, and the filtrate was neutralized with a dilute aqueous solution of hydrochloric acid and subjected to extraction with ether. The extract was washed twice with a saturated aqueous solution of sodium chloride. After evaporating the solvent, 80 ml of methanol and 0.01 g of p-toluenesulfonic acid were added and the mixture was refluxed for 2 hours in order to convert the alkyloxy fatty acid to its methyl ester. Methyl 3-n-propoxystearate was isolated by silica gel column chromatography (2.7 cm in diameter, 43 cm in length, eluent (volume ratio): n-hexane/ethyl acetate=20/1), followed by addition of 1.5 g of sodium hydroxide, 15 ml of tetrahydrofuran, 30 ml of methanol and 1 ml of water. The mixture was refluxed for 1.5 hours for hydrolysis, giving 3-n-propoxystearic acid. The yield was 72.7%.

The characteristic values of the obtained product were as follows, whereby the structure of the formula (2) wherein R¹=CH₃(CH₂)₂ is supported.
IR (NaCl, cm⁻¹):
   3300 - 2500 (OH), 2926 (CH), 2854 (CH), 1713 (C=O), 1466, 1378, 1298, 1095 (C-O-C), 938
¹NMR (CDCl₃, δ ppm):
   11.0 (1H, brs, OH), 3.70 (0.5H, quint, CH), 3.5 - 3.4 (2H, m, OCH₂), 3.36 (0.5H, m, CH), 2.6 - 2.4 (2H, m, CH₂CO₂H), 1.6 - 1.5 (2H, m, OCH₂CH₂), 1.3 - 1.2 (28H, brm, CH₂ x 14), 0.94 (3H, t, OCH₂CH₂CH₃), 0.89 (3H, t, CH₃)
melting point: 5.6°C

### Example 14

The procedure of Example 13 was repeated except that n-butanol was used in lieu of n-propanol. The yield of the obtained 3-n-butoxystearic acid was 68.5%.

The characteristic values of the obtained product were as follows, whereby the structure of the formula (2) wherein Rl=CH₃(CH₂)₃ is supported.
IR(NaCl, cm⁻¹):
   3300 - 2500 (OH), 2925 (CH), 2855 (CH), 1713 (C=O), 1466, 1378, 1295, 1099 (C-O-C), 937
¹NMR (CDCl₃, δ ppm):
   11.1 (1H, brs, OH), 3.69 (0.5H, m, CH), 3.6 - 3.5 (0.5H, m, CH; 2H, m, OCH₂), 2.6 - 2.4 (2H, m, CH₂CO₂H), 1.6 - 1.5 (2H, m, OCH₂CH₂), 1.3 - 1.2 (28H, brm, CH₂ x 14), 0.93 (3H, t, O(CH₂)₃CH₃), 0.89 (3H, t, CH₃)
melting point: -1.3°C

## Claims

1. A fatty acid derivative of the formula (1) 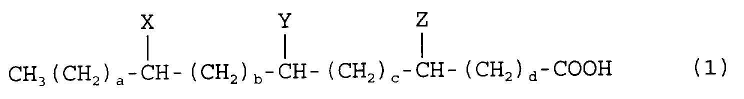 wherein each of X, Y and Z, which are the same or different, is a C₁-C₁₀ alkyloxy group or a C₃-C₁₀ cycloalkyloxy group, at least one of X, Y and Z being a C₁-C₁₀ alkyloxy group or a C₃-C₁₀ cycloalkyloxy group, each of a, b, c and d, which are the same or different, is an integer of 0 to 17 and the value of a + b + c + d is from 13 to 17; or a salt thereof.

2. A derivative as defined in claim 1 which has the formula (2) 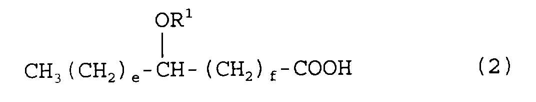 wherein R₁ is a C₁-C₁₀ alkyl group or a C₃-C₁₀ cycloalkyl group, e and f are the same or different and e is 0 or an integer of from 1 to 19, f is an integer of 1 to 19 and the value of e + f is from 15 to 19; or a salt thereof.

3. A derivative as defined in claim 1 which has the formula (3) 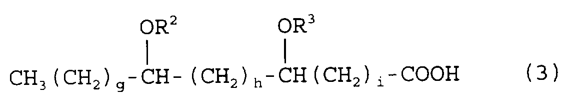 wherein each of R and R³, which are the same or different, is a C₁-C₁₀ alkyl group or a C₃-C₁₀ cycloalkyl group, each of g, h and i, which are the same or different, is 0 or an integer of 1 and the value of g + h + i is from 14 to 18; or a salt thereof.

4. A derivative as defined in claim 1 which has the formula (4) 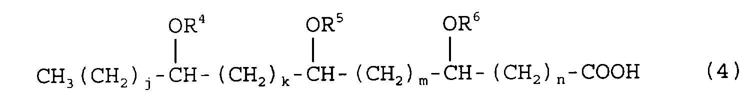 wherein each of R⁴, R⁵ and R⁶, which are the same or different, is a C₁-C₁₀ alkyl group or a C₃-C₁₀ cycloalkyl group; each of j, k, m and n, which are the same or different, is 0 or an integer of 1 to 17 and the value of j + k + m + n is from 13 to 17; or a salt thereof.

5. A derivative as defined in claim 1 which has the formula (2-p) 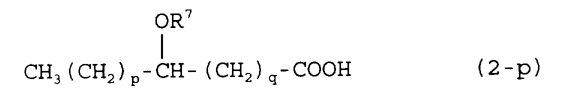 wherein R⁷ is a C₃-C₆ alkyl group or a C₅-C₇ cycloalkyl group, each of p and q, which are the same or different, is an integer of 1 to 14 and the value of p + q is 15 to 19; or a salt thereof.

6. A derivative as defined in claim 2 which is 9-methoxystearic acid, 9-ethoxystearic acid, 9-n-propyloxystearic acid, 9-iso-propyloxystearic acid, 9-n-butoxystearic acid, 9-tert-butoxystearic acid, 9-cyclohexyloxystearic acid, 10-methoxystearic acid, 10-ethoxystearic acid, 10-n-propyloxystearic acid, 10-iso-propyloxystearic acid, 10-n-butoxystearic acid, 10-tert-butoxystearic acid, 10-cyclohexyloxystearic acid, 6-methoxystearic acid, 7-methoxystearic acid, 11-methoxystearic acid, 12-methoxystearic acid, 9-methoxyeicosanoic acid, 10-methoxyeicosanoic acid, 11-methoxyeicosanoic acid, 12-methoxyeicosanoic acid, 11-methoxybehenic acid, 12-methoxybehenic acid, 13-methoxybehenic acid or 14-methoxybehenic acid; or a salt thereof.

7. A derivative as defined in claim 3 which is 9,12-dimethoxystearic acid or 10,13-dimethoxystearic acid, 9,10-dimethoxystearic acid, 9,13-dimethoxystearic acid, 10,12-dimethoxystearic acid, 10,13-dimethoxystearic acid, 11,12-dimethoxystearic acid or 11,13-dimethoxystearic acid; or a salt thereof.

8. A derivative as defined in claim 4 which is 6,9,12-trimethoxystearic acid, 6,9,13-trimethoxystearic acid, 6,10,12-trimethoxystearic acid, 6,10,13-trimethoxystearic acid, 7,9,12-trimethoxystearic acid, 7,9,13-trimethoxystearic acid, 7,10,12-trimethoxystearic acid, 7,10,13-trimethoxystearic acid, 9,12,15-trimethoxystearic acid, 9,12,16-trimethoxystearic acid, 9,13,15-trimethoxystearic acid, 9,13,16-trimethoxystearic acid, 10,12,15-trimethoxystearic acid, 10,12,16-trimethoxystearic acid, 10,13,15-trimethoxystearic acid, 10,13,16-trimethoxystearic acid, 8,11,14-trimethoxyeicosanoic acid, 8,11,15-trimethoxyeicosanoic acid, 8,12,14-trimethoxyeicosanoic acid, 8,12,15-trimethoxyeicosanoic acid, 9,11,14-trimethoxyeicosanoic acid, 9,11,15-trimethoxyeicosanoic acid, 9,12,14-trimethoxyeicosanoic acid, 9,12,15-trimethyoxyeicosanoic acid, 7,10,13-trimethoxybehenic acid, 7,10,14-trimethoxybehenic acid, 7,11,13-trimethoxybehenic acid, 7,11,14-trimethoxybehenic acid, 8,10,13-trimethoxybehenic acid, 8,10,14-trimethoxybehenic acid, 8,11,13-trimethoxybehenic acid or 8,11,14-trimethoxybehenic acid; or a salt thereof.

9. An ester which is obtainable by a process which comprises esterifying a fatty acid derivative as claimed in any one of claims 1 to 8 with an alcohol in the presence of an acid catalyst.

10. Use of a fatty acid derivative or salt thereof as claimed in any one of claims 1 to 8, or an ester as claimed in claim 9, in a cosmetic preparation or a lubricant composition.
